(19) Europäisches Patentamt
European Patent Office
Office européen des brevets

(11) EP 0 802 186 B1

(12) EUROPÄISCHE PATENTSCHRIFT

(45) Veröffentlichungstag und Bekanntmachung des Hinweises auf die Patenterteilung:
**29.11.2000 Patentblatt 2000/48**

(51) Int Cl.[7]: **C07D 213/30**, C07C 233/22, C07D 295/18, A61K 31/44, A61K 31/165

(21) Anmeldenummer: **97105721.1**

(22) Anmeldetag: **07.04.1997**

(54) **Benzyloxy-substituierte Phenylglycinolamide als Arzneimittel**

Benzyloxy-substituted phenylglycinolamides as pharmaceutical agents

Phénylglycinolamides substituées par benzyloxy comme médicaments

(84) Benannte Vertragsstaaten:
**AT BE CH DE DK ES FI FR GB GR IE IT LI LU MC NL PT SE**

(30) Priorität: **18.04.1996 DE 19615263**

(43) Veröffentlichungstag der Anmeldung:
**22.10.1997 Patentblatt 1997/43**

(73) Patentinhaber: **BAYER AG**
**51368 Leverkusen (DE)**

(72) Erfinder:
- **Goldmann, Siegfried, Dr.**
  **42327 Wuppertal (DE)**
- **Müller, Ulrich, Dr.**
  **42111 Wuppertal (DE)**
- **Connell, Richard, Dr.**
  **06516 West Haven CT (US)**
- **Bischoff, Hilmar, Dr.**
  **42113 Wuppertal (DE)**
- **Denzer, Dirk, Dr.**
  **42115 Wuppertal (DE)**
- **Gruetzmann, Rudi, Dr.**
  **42657 Solingen (DE)**
- **Beuck, Martin, Dr.**
  **40699 Erkrath (DE)**

(56) Entgegenhaltungen:
**EP-A- 0 344 519**
**EP-A- 0 716 082**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 802 186 B1

Printed by Jouve, 75001 PARIS (FR)

## Beschreibung

Die vorliegende Erfindung betrifft Benzyloxy-substituierte Phenylglycinolamide, Verfahren zu ihrer Herstellung und ihre Verwendung als Arzneimittel, insbesondere als antiatherosklerotische Arzneimittel.

EP 0 344 519 offenbart substituierte 4- (Chinolin-2-yl-methoxy) phenylessigsäure-Derivate, die in Arzneimitteln eingesetzt werden können, da sie nach oraler Gabe in Lipoxygenasesensitiven Testmodellen eine gute Wirkung zeigen.

Es ist bekannt, daß erhöhte Blutspiegel von Triglyzeriden (Hypertriglyzeridämie) und Cholesterin (Hypercholesterinämie) mit der Genese von atherosklerotischen Gefäßwand-Veränderungen und koronaren Herzkrankheiten assoziiert sind.

Ein deutlich erhöhtes Risiko für die Entwicklung koronarer Herzerkrankungen liegt darüber hinaus vor, wenn diese beiden Risikofaktoren kombiniert auftreten, was wiederum mit einer Überproduktion an Apolipoprotein B-100 einhergeht. Es besteht daher nach wie vor ein starkes Bedürfnis, wirksame Arzneimittel zur Bekämpfung der Atherosklerose sowie koronarer Herzkrankheiten zur Verfügung zu stellen.

Die vorliegende Erfindung betrifft Benzyloxy-substituierte Phenylglycinolamide der allgemeinen Formel (I)

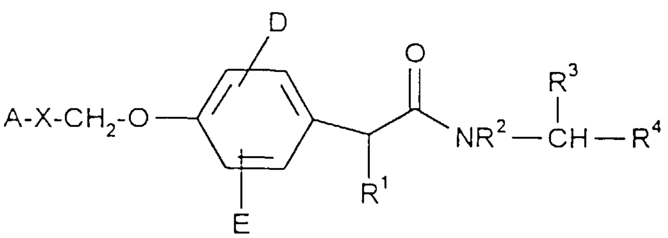

(I)

in welcher

A für einen 4- bis 8-gliedrigen, gesättigten oder partiell ungesättigten Carbocyclus steht, oder für Phenyl steht, oder für einen 5- bis 6-gliedrigen aromatischen Heterocyclus mit bis zu 3 Heteroatome aus der Reihe S, N und/ oder O steht,
wobei die oben aufgeführten Ringsysteme gegebenenfalls bis zu 5-fach gleich oder verschieden durch Phenyl, Pyridyl, Carboxyl, Cyano, Carboxyl, Halogen, Nitro, Hydroxy, durch geradkettiges oder verzweigtes Alkyl, Alkoxy, Alkoxycarbonyl, Polyfluoralkyl oder Polyfluoralkoxy mit jeweils bis zu 6 Kohlenstoffatomen oder durch eine Gruppe der Formel $-SO_2R^5$, $-NR^6R^7$ oder $-CO-NR^8R^9$ substituiert sind,
worin

$R^5$ Phenyl oder geradkettiges oder verzweigtes Alkyl mit bis zu 3 Kohlenstoffatomen bedeutet,

$R^6$, $R^7$, $R^8$ und $R^9$ gleich oder verschieden sind und Wasserstoff oder geradkettiges oder verzweigtes Alkyl mit bis zu 10 Kohlenstoffatomen bedeuten,

oder

$R^8$ und/oder $R^9$ Cycloalkyl mit 3 bis 6 Kohlenstoffatomen bedeuten, oder Benzyl oder Phenyl bedeuten, die gegebenenfalls durch Halogen, Nitro, Hydroxy oder durch geradkettiges oder verzweigtes Alkyl oder Alkoxy mit jeweils bis zu 4 Kohlenstoffatomen substituiert sind,

oder

$R^8$ und $R^9$ gemeinsam mit dem Stickstoffatom einen 5- bis 7-gliedrigen, gesättigten oder ungesättigten Heterocyclus bilden, der gegebenenfalls ein weiteres Heteroatom aus der Reihe S, N und/ oder O enthalten kann,

X für eine Bindung oder für die >C=O-Gruppe steht,

D und E gleich oder verschieden sind und für Wasserstoff, Cycloalkyl mit 3 bis 8 Kohlenstoffatomen, Azido, Hydroxy, Halogen, geradkettiges oder verzweigtes Alkyl, Alkoxy oder Alkenyl mit jeweils bis zu 6 Kohlenstoffatomen stehen,

$R^1$ für Cycloalkyl mit 3 bis 8 Kohlenstoffatomen steht, oder für geradkettiges oder verzweigtes Alkyl mit bis zu 10 Kohlenstoffatomen steht,

$R^2$ für Wasserstoff oder für geradkettiges oder verzweigtes Alkyl mit bis zu 4 Kohlenstoffatomen steht,

$R^3$ für Wasserstoff oder für die $-CH_2-OH$-Gruppe steht,

$R^4$ für Phenyl steht, das gegebenenfalls bis zu 3-fach gleich oder verschieden durch Hydroxy, Halogen oder geradkettiges oder verzweigtes Alkyl mit bis zu 5 Kohlenstoffatomen substituiert ist,

und deren Salze.

Die erfindungsgemäßen Benzyloxy-substituierten Phenylglycinolamide können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di-bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Diese Mischungen der Enantiomeren und Diastereomeren lassen sich in bekannter Weise in die stereoisomer einheitlichen Bestandteile trennen.

Ein 4- bis 8-gliedriger, gesättigter oder partiell ungesättigter Carbocyclus (A) steht im Rahmen der Erfindung für einen Cyclobuten-, Cyclopenten-, Cyclohexen-, Cyclohepten-, Cycloocten-, Cyclopropyl-, Cyclobutyl-, Cyclopentyl-, Cyclohexyl- oder Cycloheptyl-Rest. Bevorzugt sind der Cyclopenten-, Cyclohexen-, Cyclohepten-, Cyclopentyl-, Cyclohexyl- und Cycloheptyl-Rest.

Ein 5- bis 6-gliedriger aromatischer Heterocyclus (A) steht im Rahmen der Erfindung im allgemeinen beispielsweise für Thienyl, Furyl, Pyrimidyl oder Pyridyl. Bevorzugt sind Pyridyl und Thienyl.

Bevorzugt sind Verbindugen der allgemeinen Formel (I),
in welcher

A für Cyclopentyl, Cyclohexyl, Cyclopentenyl, Cyclohexenyl oder
für Pyridyl, Phenyl oder Furyl steht,
wobei die oben aufgeführten Ringe gegebenenfalls bis zu 5-fach gleich oder verschieden durch Phenyl, Pyridyl, Fluor, Chlor, Brom, Cyano, Nitro, Hydroxy, Carboxyl, durch geradkettiges oder verzweigtes Alkyl, Alkoxy, Alkoxycarbonyl, Polyfluoralkyl oder Polyfluoralkoxy mit jeweils bis zu 3 Kohlenstoffatomen oder durch eine Gruppe der Formel $-SO_2R^5$, $-NR^6R^7$ oder $-CO-NR^8R^9$ substituiert sind,
worin

$R^5$ Phenyl, Methyl oder Ethyl bedeutet,

$R^6$, $R^7$, $R^8$ und $R^9$ gleich oder verschieden sind und Wasserstoff oder geradkettiges oder verzweigtes Alkyl mit bis zu 8 Kohlenstoffatomen bedeuten,

oder

$R^8$ und/oder $R^9$ Cyclopropyl, Cyclopentyl oder Cyclohexyl bedeuten, oder Benzyl oder Phenyl bedeuten, die gegebenenfalls durch Fluor, Chlor, Brom, Nitro, Hydroxy oder durch geradkettiges oder verzweigtes Alkyl oder Alkoxy mit jeweils bis zu 3 Kohlenstoffatomen substituiert sind,

oder

$R^8$ und $R^9$ gemeinsam mit dem Stickstoffatom einen Morpholinyl-, Pyrrolidinyl-, Pyridyl- oder Piperidinylring bilden,

X für eine Bindung oder für die $>$C=O-Gruppe steht,

D und E gleich oder verschieden sind und für Wasserstoff, Cyclopropyl, Cyclopentyl, Cyclohexyl, Azido, Hydroxy, Fluor, Chlor, Brom, geradkettiges oder verzweigtes Alkyl, Alkoxy oder Alkenyl mit jeweils bis zu 3 Kohlenstoffatomen stehen,

$R^1$ für Cyclopropyl, Cyclobutyl, Cyclopentyl, Cyclohexyl oder Cycloheptyl steht, oder für geradkettiges oder verzweigtes Alkyl mit bis zu 8 Kohlenstoffatomen steht,

$R^2$ für Wasserstoff, oder für geradkettiges oder verzweigtes Alkyl mit bis zu 3 Kohlenstoffatomen steht,

$R^3$ für Wasserstoff oder für die $-CH_2-OH$-Gruppe steht,

$R^4$ für Phenyl steht, das gegebenenfalls bis zu 2-fach gleich oder verschieden durch Hydroxy, Fluor, Chlor, Brom oder geradkettiges oder verzweigtes Alkyl mit bis zu 4 Kohlenstoffatomen substituiert ist,

und deren Salze.

Besonders bevorzugt sind Verbindungen der allgemeinen Formel (I), in welcher

A für Cyclopentyl, Cyclohexyl, Cyclopentenyl, Cyclohexenyl oder für Phenyl oder Pyridyl steht, wobei die oben aufgeführten Ringe gegebenenfalls bis zu 3-fach gleich oder verschieden durch Fluor, Chlor, Brom, Cyano, Nitro, Hydroxy, durch geradkettiges oder verzweigtes Alkyl, Alkoxy, Alkoxycarbonyl mit jeweils bis zu 3 Kohlenstoffatomen, Trifluormethyl, Trifluormethoxy, Carboxyl, oder durch eine Gruppe der Formel $-SO_2R^5$, $-NR^6R^7$ oder $-CO-NR^8R^9$ substituiert sind,
worin

$R^5$ Phenyl oder Methyl bedeutet,

$R^6$, $R^7$, $R^8$ und $R^9$ gleich oder verschieden sind und Wasserstoff oder geradkettiges oder verzweigtes Alkyl mit bis zu 6 Kohlenstoffatomen bedeuten,

oder

$R^8$ und/oder $R^9$ Cyclopropyl, Cyclopentyl oder Cyclohexyl bedeuten, oder Benzyl oder Phenyl bedeuten, die gegebenenfalls durch Fluor, Chlor, Brom, Nitro, Hydroxy, Methyl oder Methoxy substituiert sind,

oder

$R^8$ und $R^9$ gemeinsam mit dem Stickstoffatom einen Morpholinyl-, Pyrrolidinyl- oder Piperidinylring bilden,

X für eine Bindung oder für die $>$C=O-Gruppe steht,

D und E gleich oder verschieden sind und für Wasserstoff, Fluor, Chlor oder Brom stehen,

$R^1$ für Cyclopropyl, Cyclobutyl, Cyclopentyl, Cyclohexyl oder Cycloheptyl steht, oder für geradkettiges oder

verzweigtes Alkyl mit bis zu 8 Kohlenstoffatomen steht,

$R^2$ für Wasserstoff steht,

$R^3$ für Wasserstoff oder für die $-CH_2$-OH-Gruppe steht,

$R^4$ für Phenyl steht, das gegebenenfalls bis zu 2-fach gleich oder verschieden durch Hydroxy, Fluor, Chlor, Brom oder geradkettiges oder verzweigtes Alkyl mit bis zu 3 Kohlenstoffatomen substituiert ist,

und deren Salze.

Ganz besonders bevorzugt sind Verbindungen der allgemeinen Formel (I), in welcher

D und E für Wasserstoff stehen

und

$R^1$ für Cyclopentyl, Cyclohexyl oder Cycloheptyl steht.

Außerdem wurde ein Verfahren zur Herstellung der erfindungsgemäßen Verbindungen der allgemeinen Formel (I) gefunden, dadurch gekennzeichnet, daß man

Carbonsäuren der allgemeinen Formel (II),

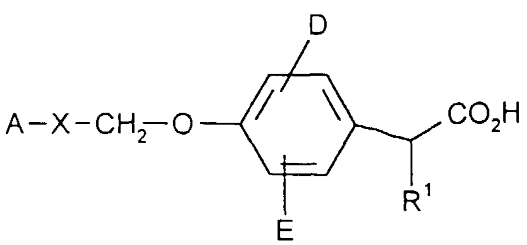 (II)

in welcher

A, D, E, X und $R^1$ die angegebene Bedeutung haben,

gegebenenfalls unter vorgeschalteter Aktivierung der Carbonsäurefunktion mit Verbindungen der allgemeinen Formel (III)

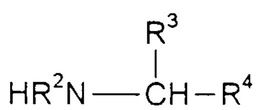 (III)

in welcher

$R^2$, $R^3$ und $R^4$ die angegebene Bedeutung haben,

gegebenenfalls unter Schutzgasatmosphäre, gegebenenfalls in inerten Lösemitteln, in Anwesenheit einer Base und/oder Hilfsmittels umsetzt.

Das erfindungsgemäße Verfahren kann durch folgendes Formelschema beispielhaft erläutert werden:

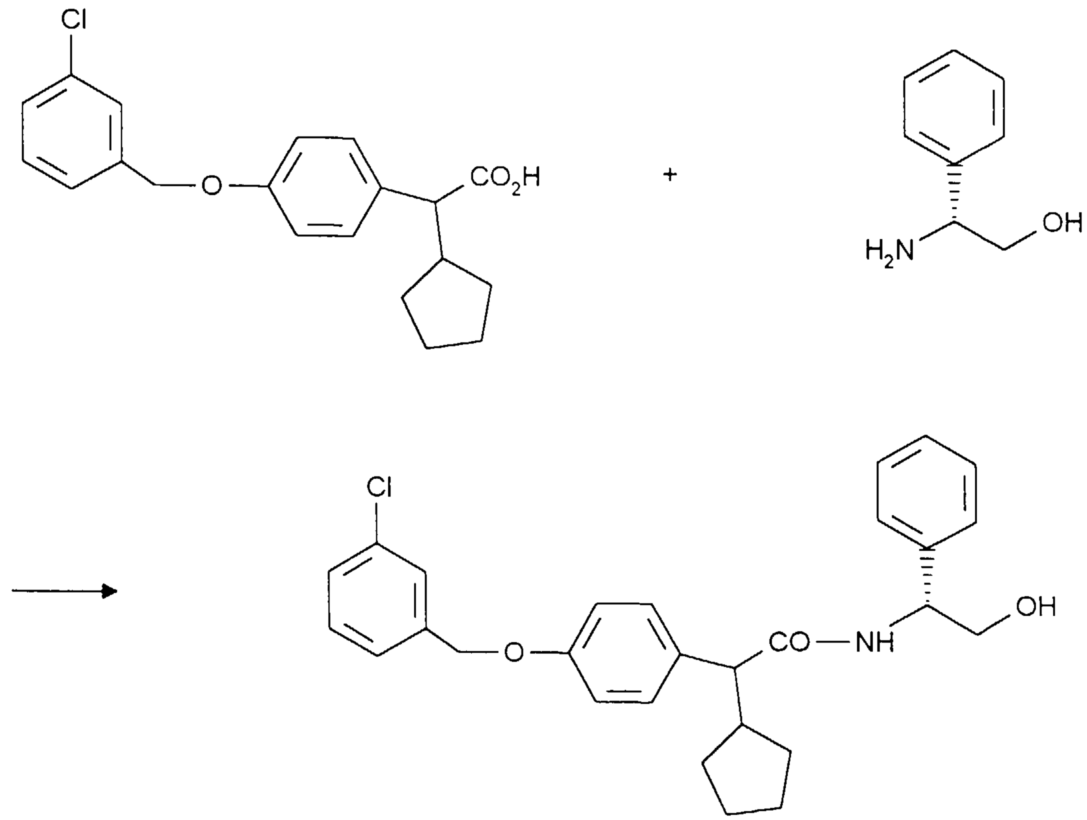

Als Lösemittel eignen sich hierbei inerte organische Lösemittel, die sich unter den Reaktionsbedingungen nicht verändern. Hierzu gehören Ether, wie Diethylether oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohenwasserstoffe wie Benzol, Xylol,Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Dichlormethan, Tetrahydrofuran, Aceton und Dimethylformamid.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliumethanolat, oder Natrium- oder Kaliummethanolat, oder organische Amine wie Triethylamin, Picolin oder N-Methylpiperidin, oder Amide wie Natriumamid oder Lithiumdiisopropylamid, oder metallorganische Verbindungen wie Butyllithium oder Phenyllithium. Bevorzugt sind Natrium- und Kaliumcarbonat und Triethylamin.

Die Base wird in einer Menge von 0,6 mol bis 5 mol, bevorzugt von 0,7 mol bis 2 mol, bezogen auf 1 mol der Verbindung der allgemeinen Formel (II) eingesetzt.

Die Reaktion wird im allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im allgemeinen arbeitet man bei Normaldruck.

Zur Aktivierung der Carbonsäurefunktion eignen sich im allgemeinen Basen und/oder Dehydratisierungsreagenzien wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphonsäureanhydrid oder Isobutylchloroformat oder Benzotriazolyloxy-tris-(dimethylamino)phosphonium-hexafluorophosphat oder Phosphorsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid.

Die säurebindenden Mittel und Dehydratisierungsreagenzien werden im allgemeinen in einer Menge von 0,5 bis 3 mol, bevorzugt von 1 bis 1,5 mol, bezogen auf 1 mol der entsprechenden Carbonsäuren, eingesetzt.

Die Verbindungen der allgemeinen Formel (II) sind teilweise bekannt oder neu und können dann beispielsweise hergestellt werden, indem man

Verbindungen der allgemeinen Formel (IV)

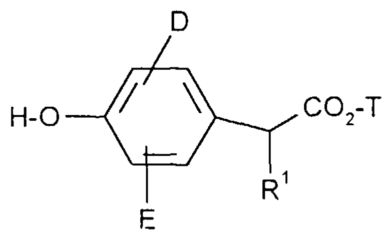

(IV)

in welcher

D, E und $R^1$ die angegebene Bedeutung haben,

und

T für eine typische Hydroxyschutzgruppe, bevorzugt für Benzyl oder tert.-Butyl steht,

nach Abspaltung dieser Schutzgruppe nach üblichen Methoden,
mit Verbindungen der allgemeinen Formel (V)

$$A\text{-}X\text{-}CH_2\ Y \qquad (V)$$

in welcher

A und X die oben angegebene Bedeutung haben

und

Y für Halogen, vorzugsweise Brom steht,

in inerten Lösemitteln, gegebenenfalls in Anwesenheit einer Base umsetzt,
und im Fall der Säuren die Ester verseift.

Die Verbindungen der allgemeinen Formeln (IV) und (V) sind an sich bekannt oder nach üblichen Methoden herstellbar.

Die Verbindungen der allgemeinen Formel (III) sind ebenfalls bekannt oder nach üblichen Methoden herstellbar.

Die erfindungsgemäßen Verbindungen der allgemeinen Formel (I) haben ein nicht vorhersehbares pharmakologisches Wirkspektrum.

Sie können als Wirkstoffe in Arzneimitteln zur Reduzierung von Veränderungen an Gefäßwänden Verwendung finden und zur Behandlung von Koronaren Herzerkrankungen, Herzinsuffizienz, Störungen der Hirnleistung, ischämischen Gehirnerkrankungen, Apoplex, Durchblutungsstörungen, Mikrozirkulationsstörungen und Thrombosen.

Weiterhin spielt bei der Okklusion von Gefäßen die Proliferation glatter Muskelzellen eine ausschlaggebende Rolle. Die erfindungsgemäßen Verbindungen sind geeignet, diese Proliferation zu inhibieren und damit atherosklerotische Prozesse zu verhindern.

Die erfindungsgemäßen Verbindungen zeichnen sich durch eine Senkung der ApoB-100-assoziierten Lipoproteinen (VLDL und seiner Abbauprodukte, wie z.B. LDL), des ApoB-100, der Triglyceride und des Cholesterins aus. Damit besitzen sie wertvolle, im Vergleich zum Stand der Technik überlegene pharmakologische Eigenschaften.

Überraschenderweise besteht die Wirkung der erfindungsgemäßen Verbindungen zunächst in einer Verminderung oder vollständigen Inhibierung der Bildung und/oder der Freisetzung von ApoB-100-assoziierten Lipoproteinen aus Leberzellen, was eine Senkung des VLDL-Plasmaspiegels zur Folge hat. Diese VLDL-Senkung muß mit einer Senkung der Plasmaspiegel von ApoB-100, LDL, Triglyceriden und von Cholesterin einhergehen; es werden also gleichzeitig mehrere der obengenannten Risikofaktoren gesenkt, die an Gefäßwandveränderungen beteiligt sind.

Die erfindungsgemäßen Verbindungen können daher zur Präventation und Behandlung von Atherosklerose, der Fettsucht, Pankreatitis und der Obstipation eingesetzt werden.

### 1. Hemmung der Freisetzung ApoB-100-assoziierter Lipoproteine

Der Test zum Nachweis der Hemmung der Freisetzung ApoB-100-assoziierter Liproproteine aus Leberzellen erfolgte in vitro mit kultivierten Leberzellen, bevorzugt mit Zellen der humanen Linie HepG2. Diese Zellen werden unter Standardbedingungen in Medium für die Kultur eukariontischer Zellen gezüchtet, bevorzugt in RPMI 1640 mit 10% fötalem Kälberserum. HepG2-Zellen synthetisieren und sezernieren in den Kulturüberstand ApoB-100-assoziierte Lipoproteinpartikel, die im Prinzip ähnlich aufgebaut sind wie die VLDL- bzw. LDL-Partikel, die im Plasma zu finden sind.

Diese Partikel können mit einem Immunoassay für humanes LDL nachgewiesen werden. Dieser Immunoassay erfolgt mit Antikörpern, die im Kaninchen gegen humanes LDL unter Standardbedingungen induziert worden waren. Die anti-LDL-Antikörper (Kan-anti-LDL-Ak) wurden an einem Immunosorbens mit humanem LDL affinitätschromatographisch gereinigt. Diese gereinigten Kan-anti-LDL-Ak werden an die Oberfläche von Plastik adsorbiert. Zweckmäßigerweise erfolgt diese Adsorbtion an die Plastikoberfläche von Mikrotitierplatten mit 96 Vertiefungen, bevorzugt an MaxiSorp-Platten. Wenn im Überstand von Hep-G2-Zellen ApoB-100-assoziierte Partikel vorhanden sind, dann können diese an die insolubilisierten Kan-anti-LDL-Ak binden, und es entsteht ein Immunkomplex, der an die Plastikoberfläche gebunden ist. Nicht gebundene Proteine werden durch Waschen entfernt. Der sich an der Plastikoberfläche befindliche Immunkomplex wird mit monoklonalen Antikörpern nachgewiesen, die nach Standardbedingungen gegen humanes LDL induziert und gereinigt worden waren. Diese Antikörper wurden mit dem Enzym Peroxidase konjugiert. Peroxidase setzt das farblose Substrat TMB in Gegenwart von $H_2O_2$ in ein gefärbtes Produkt um. Nach Ansäuerung des Reaktionsgemisches mit $H_2SO_4$ wird die spezifische Lichtadsorption bei 450 nm bestimmt, die ein Maß für die Menge von ApoB-100-assoziierten Partikeln ist, die von den HepG2-Zellen in den Kulturüberstand sezerniert worden waren.

Überraschenderweise hemmen die erfindungsgemäßen Verbindungen die Freisetzung der ApoB-100-assoziierten Partikel. Der $IC_{50}$-Wert gibt an, bei welcher Substanzkonzentration die Lichtadsorption im Vergleich zur Kontrolle (Lösemittelkontrolle ohne Substanz) um 50% inhibiert ist.

### 2. Bestimmung der VLDL-Sekretion in vivo am Hamster

Der Effekt der Testsubstanzen auf die VLDL-Sekretion in vivo wird am Hamster untersucht. Hierzu werden Goldhamster nach Prämedikation mit Atropin (83 mg/kg s.c.) mit Ketavet (83 mg/kg s.c.) und Nembutal (50 mg/kg i.p.) narkotisiert. Wenn die Tiere reflexfrei geworden sind, wird die V. jugularis freipräpariert und kanüliert. Anschließend werden 0,25 ml/kg einer 20%igen Lösung von Triton WR-1339 in physiologischer Kochsalzlösung appliziert. Dieses Detergens hemmt die Lipoproteinlipase und führt so zu einem Anstieg des Triglyceridspiegels aufgrund eines ausbleibenden Katabolismus von sezernierten VLDL-Partikeln. Dieser Triglyceridanstieg kann als Maß für die VLDL-Sekretionsrate herangezogen werden. Den Tieren wird vor sowie ein und zwei Stunden nach Applikation des Detergens durch Punktion des retroorbitalen Venenplexus Blut entnommen. Das Blut wird zwei Stunden bei Raumtemperatur, anschließend über Nacht bei 4°C inkubiert, um die Gerinnung vollständig abzuschließen. Danach wird 5 Minuten bei 10.000 g zentrifugiert. Im so erhaltenen Serum wird die Triglyceridkonzentration mit Hilfe eines modifizierten kommerziell erhältlichen Enzymtests bestimmt (Merckotest® Triglyceride Nr. 14354). 100 µl Serum werden mit 100 µl Testreagenz in 96-Lochplatten versetzt und 10 Minuten bei Raumtemperatur inkubiert. Anschließend wird die optische Dichte bei einer Wellenlänge von 492 nm in einem automatischen Platten-Lesegerät bestimmt (SLT-Spectra). Serumproben mit einer zu hohen Triglyceridkonzentration werden mit physiologischer Kochsalzlösung verdünnt. Die in den Proben enthaltene Triglyceridkonzentration wird mit Hilfe einer parallel gemessenen Standardkurve bestimmt. Testsubstanzen werden in diesem Modell entweder unmittelbar vor Applikation des Detergens intravenös verabreicht oder vor Einleitung der Narkose oral oder subkutan.

### 3. Hemmung der intestinalen Triglyceridabsorption in vivo (Ratten)

Die Substanzen, die auf ihre triglyceridabsorptionshemmende Wirkung in vivo untersucht werden sollen, werden männlichen Wistar-Ratten mit einem Körpergewicht zwischen 170 und 230 g oral verabreicht. Zu diesem Zweck werden die Tiere 18 Stunden vor der Substanzapplikation in Gruppen zu 6 Tieren eingeteilt und anschließend wird ihnen das Futter entzogen. Trinkwasser steht den Tieren ad libitum zur Verfügung. Die Tiere der Kontrollgruppen erhalten eine wäßrige Traganth-Suspension bzw. eine Traganth-Suspension die Olivenöl enthält. Die Traganth-Olivenöl-Suspension wird mit dem Ultra-Turrax hergestellt. Die zu untersuchenden Substanzen werden in einer entsprechenden Traganth-Olivenöl-Suspension ebenfalls mit dem Ultra-Turrax, direkt vor der Substanzapplikation suspendiert.

Jeder Ratte wird vor der Schlundsondenapplikation zur Bestimmung des basalen Serumtriglyceridgehaltes

Blut durch Punktion des retroorbitalen Venenplexus' entnommen. Anschließend werden die Traganth-Suspension, die Traganth-Olivenöl-Suspensionen ohne Substanz (Kontrolltiere), bzw. die Substanzen, suspendiert in einer entsprechenden Traganth-Olivenöl-Suspension, den nüchternen Tieren mit einer Schlundsonde verabreicht. Die weiteren Blutentnahmen zur Bestimmung des postprandialen Serumtriglyceridanstiegs erfolgen in der Regel 1, 2 und 3 Stunden nach der Schlundsondenapplikation.

Die Blutproben werden zentrifugiert und nach Gewinnung des Serums die Triglyceride photometrisch mit einem EPOS-Analyzer 5060 (Eppendorf Gerätebau, Netheler & Hinz GmbH, Hamburg) bestimmt. Die Bestimmung der Trigylceride erfolgt vollenzymatisch mit einem handelsüblichen UV-Test.

Der postprandiale Serumtriglyceridanstieg wird durch Subtraktion des Triglyceridvorwertes jeden Tieres von seinen korrespondierenden postprandialen Triglyceridkonzentrationen (1, 2 und 3 Stunden nach Applikation) ermittelt.

Die Differenzen (in mmol/l) zu jedem Zeitpunkt (1, 2 und 3 Stunden) werden in den Gruppen gemittelt, und die Mittelwerte des Serumtriglyceridanstiegs ($\Delta$TG) der substanzbehandelten Tiere mit den Tieren verglichen, die nur die Traganth-Öl-Suspension erhielten.

Ebenso wird der Serumtriglyceridverlauf der Kontrolltiere, die nur Traganth erhielten, berechnet. Der Substanzeffekt zu jedem Zeitpunkt (1, 2 oder 3 Stunden) wird wie folgt ermittelt und in $\Delta$% von der ölbelasteten Kontrolle angegeben.

$$\Delta\%\ \text{Triglyceridanstieg} = \frac{\Delta TG_{Substanz} - \Delta TG_{Traganthkontrolle}}{\Delta TG_{Ölbelastung} - \Delta TG_{Traganthkontrolle}} \times 100$$

Effekt von 10 mg Prüfsubstanz / kg KG p.o. auf den Triglyceridanstieg ($\Delta$%) 2 h nach einer Triglyceridbelastung im Serum nüchterner Ratten. Der Serumtriglyceridanstieg fettbelasteter Kontrolltiere bezogen auf den Serumtriglyceridspiegel von Traganth-Kontrolltieren entspricht 100%. n = 6 Tiere pro Gruppe.

Die statistische Auswertung erfolgt mit Student's t-Test nach vorheriger Überprüfung der Varianzen auf Homogenität.

Substanzen, die zu einem Zeitpunkt den postprandialen Serumtriglyceridanstieg, verglichen mit dem der unbehandelten Kontrollgruppe, statistisch signifikant ($p < 0{,}05$) um mindestens 30 % vermindern, werden als pharmakologisch wirksam angesehen.

**4. Hemmung der VLDL-Sekretion in vivo (Ratte)**

Die Wirkung der Testsubstanzen auf die VLDL-Sekretion wird ebenfalls an der Ratte untersucht. Dazu wird Ratten 500 mg/kg Körpergewicht Triton WR-1339 (2,5 mg/kg), gelöst in physiologischer Kochsalzlösung, intravenös in die Schwanzvene appliziert. Triton WR-1339 inhibiert die Lipoproteinlipase und führt somit durch Hemmung des VLDL-Katabolismus zu einem Anstieg des Triglycerid- und Cholsterinspiegels. Diese Anstiege können als Maß für die VLDL-Sekretionsrate herangezogen werden.

Den Tieren wird vor sowie eine und zwei Stunden nach Applikation des Detergens durch Punktion des retroorbitalen Venenplexus Blut entnommen. Das Blut wird zur Gerinnung 1 h bei Raumtemperatur inkubiert und das Serum durch Zentrifugation mit 10 000 g für 20 s gewonnen. Anschließend werden die Triglyceride mittels eines handelsüblichen gekoppelten Enzymtests (Sigma Diagnostics®, Nr. 339) bei einer Wellenlänge von 540 nm photometrisch bestimmt. Die Messung erfolgt mit Hilfe eines ebenfalls gekoppelten Enzymtests (Boehringer Mannheim®, Nr. 1442350) bei einer Wellenlänge von 546 nm. Proben mit Triglycerid- bzw. Cholesterinkonzentrationen, die den Meßbereich der Methoden überschreiten, werden mit physiologischer Kochsalzlösung verdünnt. Die Ermittlung der jeweiligen Serumkonzentrationen erfolgt anhand parallel gemessener Standardreihen. Testsubstanzen werden unmittelbar nach der Tritoninjektion oral, intravenös oder subcutan appliziert.

Die Erfindung betrifft außerdem die Kombination von Benzyloxy-substituierten Phenylglycinolamiden der allgemeinen Formel (I) mit einem Glucosidase- und/oder Amylasehemmer zur Behandlung von familiärer Hyperlipidaemien, der Fettsucht (Adipositas) und des Diabetes mellitus. Glucosidase- und/oder Amylasehemmer im Rahmen der Erfindung sind beispielsweise Acarbose, Adiposine, Voglibose, Miglitol, Emiglitate, MDL-25637, Camiglibose (MDL-73945), Tendamistate, AI-3688, Trestatin, Pradimicin-Q und Salbostatin.

Bevorzugt ist die Kombination von Acarbose, Miglitol, Emiglitate oder Voglibose mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

<u>**Ausgangsverbindungen**</u>

**Beispiel I**

2-[4-(3-Chlorbenzyloxyphenyl)-2-cyclopentyl]-essigsäure-tert.butylester

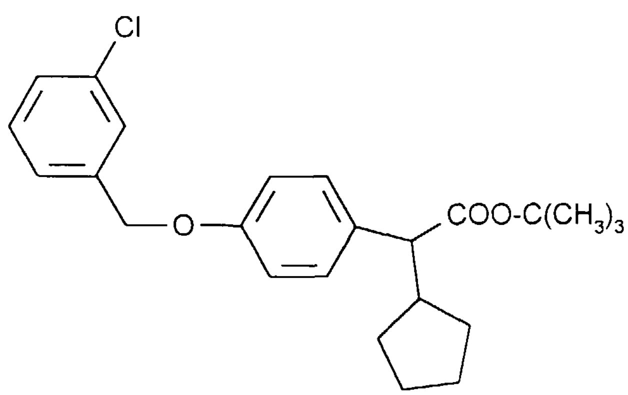

1 g 2-(4-Hydroxyphenyl)-2-cyclopentyl-essigsäure-tert.butylester (US 834.734) und 1,2 ml 3-Chlorbenzylbromid werden in 10 ml DMF gelöst und mit 0,9 g $K_2CO_3$ 14 h auf 60-70°C erwärmt. Nach Abkühlung wird mit Wasser versetzt und mit Ether ausgeschüttelt und getrocknet. Man erhält 1,3 g der Titelverbindung als Öl. $R_f$ = 0,48 (Cyclohexan / Essigester 9:1)

**Beispiel II**

2-[4-(3-Chlorbenzyloxyphenyl)-2-cyclopentyl]-essigsäure

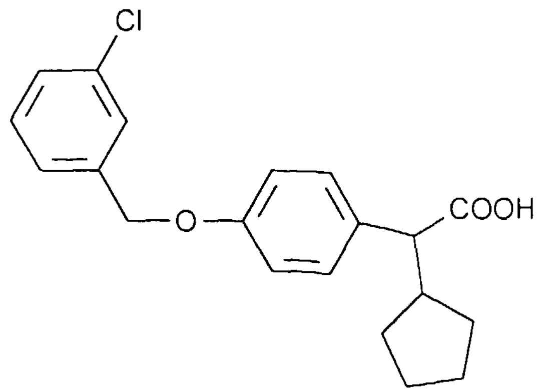

1,1 g der Verbindung aus Beispiel I werden in 12 ml Dioxan gelöst und mit 0,6 ml konz. HCl versetzt, 8 h am Rückfluß gekocht, eingeengt, in $CH_2Cl_2$ gelöst, mit Wasser neutral gewaschen und eingeengt. Der Rückstand wird mit $H_2O$ kristallisiert.
Ausbeute: 0,6 g (60% d.Th.)
Fp.: 99-100°C

**Herstellungsbeispiele**

**Beispiel 1**

2-[4-(3-Chlorbenzyloxyphenyl)-2-cyclopentyl]-N-[(2-hydroxy)-1-(R)-phenylethyl]-2-essigsäureamid

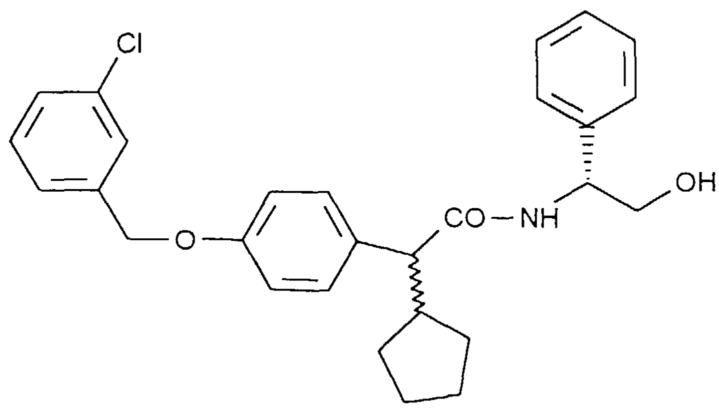

0,52 g der Verbindung aus Beispiel II werden in 8 ml THF gelöst, auf - 30°C gekühlt und nacheinander mit 0,62 ml Triethylamin und 0,13 ml Mesylchlorid versetzt und 30 min bei -30°C gerührt. Anschließend werden 0,25 g (R)-(-)-2-Hydroxy-1-phenyl-ethylamin und Dimethylaminopyridin (beide gelöst in 4 ml THF) bei -30°C zugetropft, 30 min bei -30°C und 2 h bei Raumtemperatur gerührt. Man erhält 50% der Titelverbindung. Fp.. 148-149°C
In Analogie zur Vorschrift des Beispiels 1 werden die in der Tabelle 1 aufgeführten Verbindungen hergestellt:

**Tabelle 1**

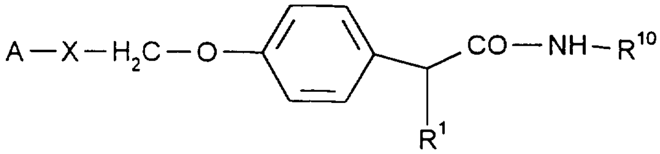

| Bsp.-Nr. | A | X | $R^1$ | $R^{10}$ | Mp. (°C) | $R_f$ * |
|---|---|---|---|---|---|---|
| 2 | 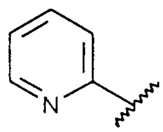 | Bin-dung | 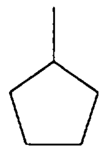 | 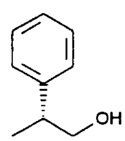 | 145-7 | |
| 3 | 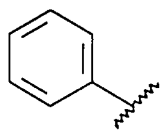 | Bin-dung | 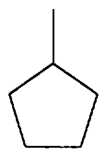 | 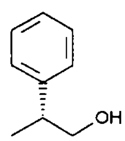 | 179-80 | |
| 4 | 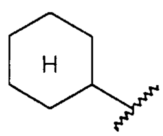 | Bin-dung | 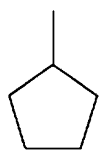 | 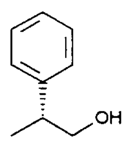 | 155-6 | |
| 5 | 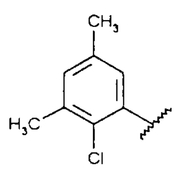 | 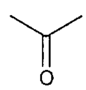 | 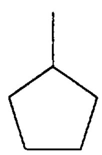 | 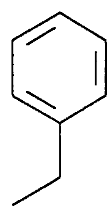 | amorph | 0,5 [1)] |
| 6 | 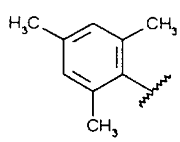 | 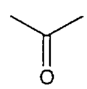 | 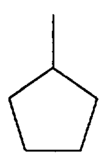 | 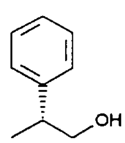 | amorph | 0,25 und 0,31 [1)] |
| 7 | 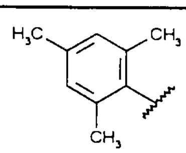 | 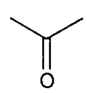 | 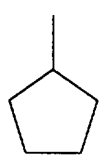 | 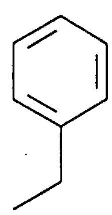 | amorph | 0,34 [2)] |
| 8 | 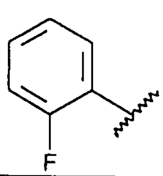 | Bin-dung | 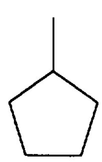 | 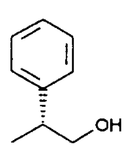 | 167 | |

| Bsp.-Nr. | A | X | $R^1$ | $R^{10}$ | Mp. (°C) | $R_f$ * |
|---|---|---|---|---|---|---|
| 9 | 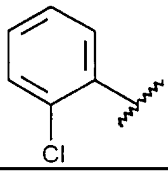 | Bin-dung | 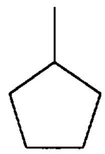 | 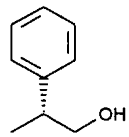 | 139-40 | |
| 10 | 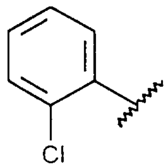 | Bin-dung | 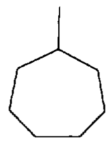 | 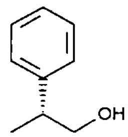 | amorph | 0,48 und 0,40 [1)] |
| 11 | 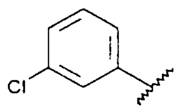 | Bin-dung | 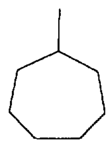 | 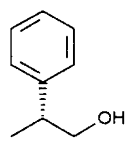 | 156-7 | |
| 12 | 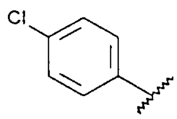 | Bin-dung | 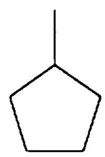 | 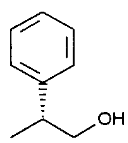 | 165-6 | |
| 13 | 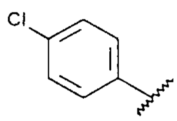 | Bin-dung | 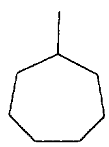 | 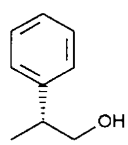 | 175-6 | |
| 14 | 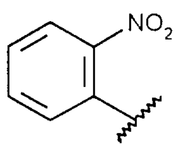 | Bin-dung | 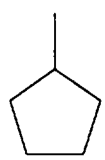 | 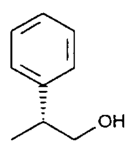 | 152-3 | |
| 15 | 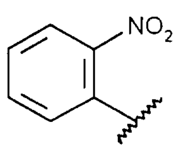 | Bin-dung | 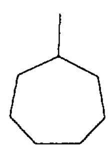 | 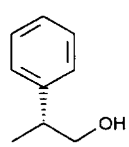 | amorph | 0,37 und 0,33 [1)] |
| 16 | 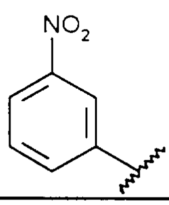 | Bin-dung | 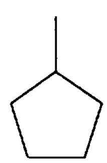 | 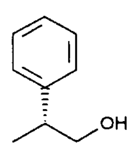 | 144-5 | |
| 17 | 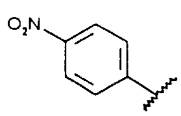 | Bin-dung | 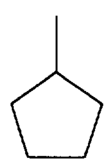 | 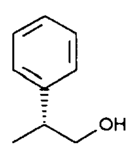 | 160-3 | |

| Bsp.-Nr. | A | X | $R^1$ | $R^{10}$ | Mp. (°C) | $R_f$ * |
|---|---|---|---|---|---|---|
| 18 | 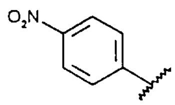 | Bin-dung | 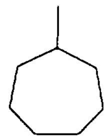 | 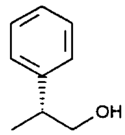 | amorph | |
| 19 | 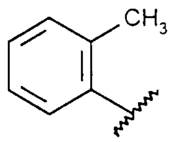 | Bin-dung | 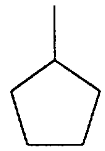 | 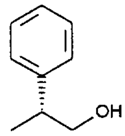 | 144-6 | |
| 20 | 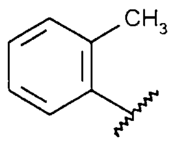 | Bin-dung | 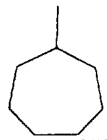 | 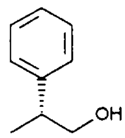 | 132-4 | |
| 21 | 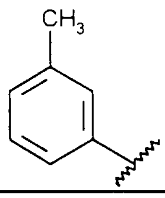 | Bin-dung | 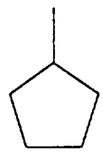 | 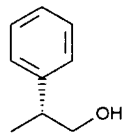 | 151-3 | |
| 22 | 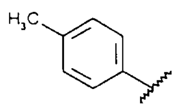 | Bin-dung | 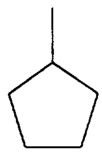 | 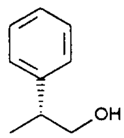 | 175-7 | |
| 23 | 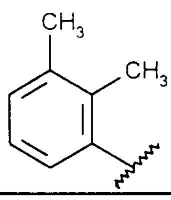 | Bin-dung | 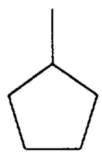 | 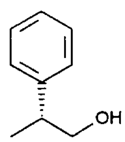 | 144 | |
| 24 | 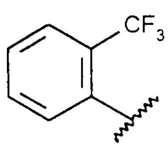 | Bin-dung | 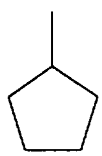 | 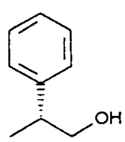 | 160-2 | |
| 25 | 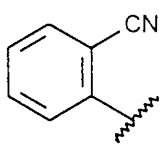 | Bin-dung | 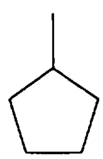 | 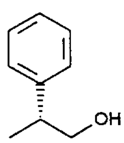 | 158-9 | |
| 26 | 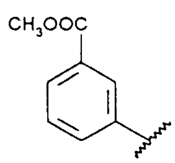 | Bin-dung | 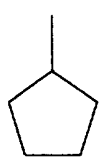 | 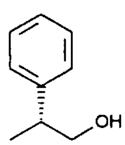 | 156-7 | |

| Bsp.-Nr. | A | X | $R^1$ | $R^{10}$ | Mp. (°C) | $R_f$ * |
|---|---|---|---|---|---|---|
| 27 | 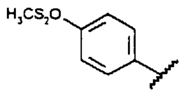 | Bin-dung | 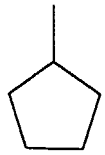 | 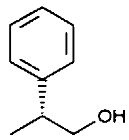 | amorph | 0,10 [1)] |
| 28 | 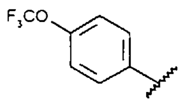 | Bin-dung | 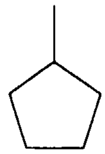 | 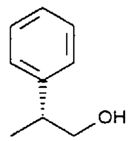 | 186-8 | |
| 29 | 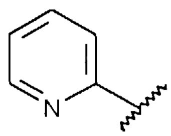 | Bin-dung | 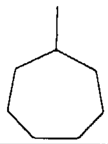 | 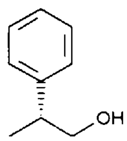 | | 0,19 [1)] |
| 30 | 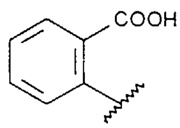 | Bin-dung | 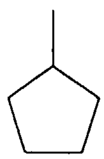 | 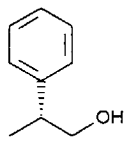 | 182-3 | |
| 31 | 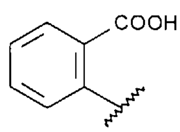 | Bin-dung | 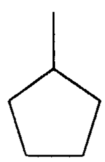 | 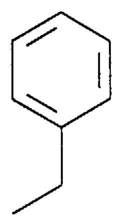 | 177-8 | |
| 32 | 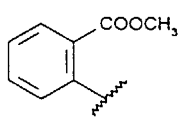 | Bin-dung | 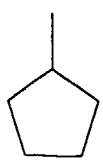 | 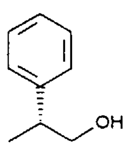 | 152-3 | |
| 33 | 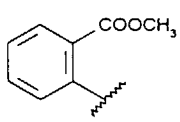 | Bin-dung | 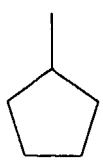 | 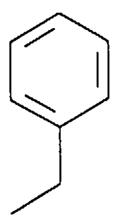 | 115-6 | |
| 34 | 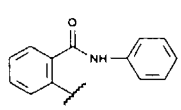 | Bin-dung | 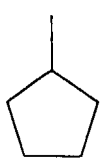 | 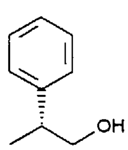 | amorph | 0,67 [1)] |

| Bsp.-Nr. | A | X | $R^1$ | $R^{10}$ | Mp. (°C) | $R_f$ * |
|---|---|---|---|---|---|---|
| 35 | 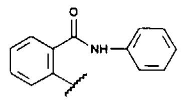 | Bindung | 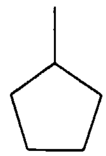 | 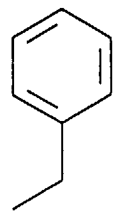 | amorph | 0,29 [2] |
| 36 | 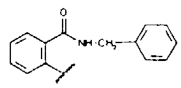 | Bindung | 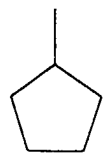 | 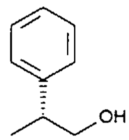 | amorph | 0,22 [1] |
| 37 | 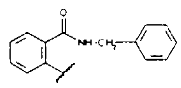 | Bindung | 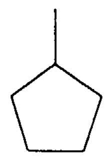 | 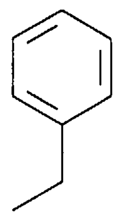 | 165-6 | |
| 38 | 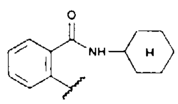 | Bindung | 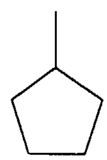 | 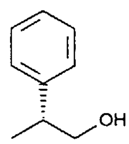 | amorph | 0,26 [1] |
| 39 | 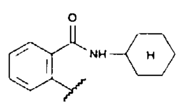 | Bindung | 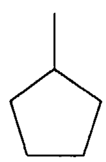 | 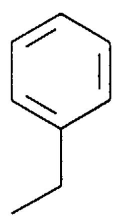 | 157-8 | |
| 40 | 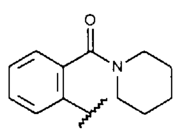 | Bindung | 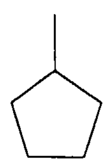 | 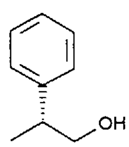 | amorph | 0,13 [1] |
| 41 | 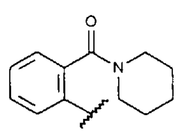 | Bindung | 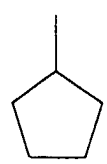 | 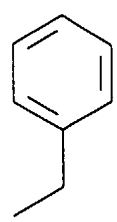 | amorph | 0,08 [2]<br>0,30 [1] |

| Bsp.-Nr. | A | X | $R^1$ | $R^{10}$ | Mp. (°C) | $R_f$ * |
|---|---|---|---|---|---|---|
| 42 | 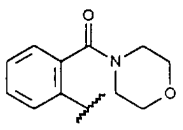 | Bindung | 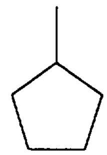 | 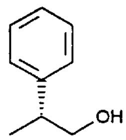 | amorph | 0,04 1) |
| 43 | 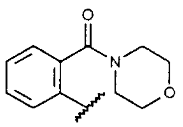 | Bindung | 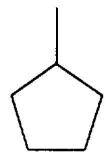 | | amorph | |

1) Cyclohexan / EE = 1:1
2) Cyclohexan / EE = 7:3

## Patentansprüche

**1.** Benzyloxy-substituierte Phenylglycinolamide der allgemeinen Formel (I)

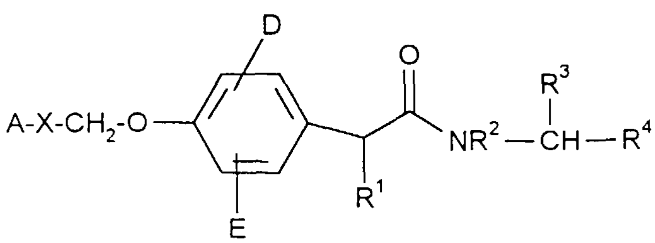 (I)

in welcher

A für einen 4- bis 8-gliedrigen, gesättigten oder partiell ungesättigten Carbocyclus steht, oder für Phenyl steht, oder für einen 5- bis 6-gliedrigen aromatischen Heterocyclus mit bis zu 3 Heteroatome aus der Reihe S, N und/oder O steht, wobei die oben aufgeführten Ringsysteme gegebenenfalls bis zu 5-fach gleich oder verschieden durch Phenyl, Pyridyl, Carboxyl, Cyano, Carboxyl, Halogen, Nitro, Hydroxy, durch geradkettiges oder verzweigtes Alkyl, Alkoxy, Alkoxycarbonyl, Polyfluoralkyl oder Polyfluoralkoxy mit jeweils bis zu 6 Kohlenstoffatomen oder durch eine Gruppe der Formel $-SO_2R^5$, $-NR^6R^7$ oder $-CO-NR^8R^9$ substituiert sind, worin

$R^5$ Phenyl oder geradkettiges oder verzweigtes Alkyl mit bis zu 3 Kohlenstoffatomen bedeutet,

$R^6$, $R^7$, $R^8$ und $R^9$ gleich oder verschieden sind und Wasserstoff oder geradkettiges oder verzweigtes Alkyl mit bis zu 10 Kohlenstoffatomen bedeuten,

oder

$R^8$ und/oder $R^9$ Cycloalkyl mit 3 bis 6 Kohlenstoffatomen bedeuten, oder Benzyl oder Phenyl bedeuten, die gegebenenfalls durch Halogen, Nitro, Hydroxy oder durch geradkettiges oder verzweigtes Alkyl oder Alkoxy mit jeweils bis zu 4 Kohlenstoffatomen substituiert sind,

oder

$R^8$ und $R^9$ gemeinsam mit dem Stickstoffatom einen 5- bis 7-gliedrigen, gesättigten oder ungesättigten Heterocyclus bilden, der gegebenenfalls ein weiteres Heteroatom aus der Reihe S, N und/oder O enthalten kann,

X für eine Bindung oder für die >C=O-Gruppe steht,

D und E gleich oder verschieden sind und für Wasserstoff, Cycloalkyl mit 3 bis 8 Kohlenstoffatomen, Azido, Hydroxy, Halogen, geradkettiges oder verzweigtes Alkyl, Alkoxy oder Alkenyl mit jeweils bis zu 6 Kohlenstoffatomen stehen,

$R^1$ für Cycloalkyl mit 3 bis 8 Kohlenstoffatomen steht, oder für geradkettiges oder verzweigtes Alkyl mit bis zu 10 Kohlenstoffatomen steht,

$R^2$ für Wasserstoff oder für geradkettiges oder verzweigtes Alkyl mit bis zu 4 Kohlenstoffatomen steht,

$R^3$ für Wasserstoff oder für die $-CH_2-OH$-Gruppe steht,

$R^4$ für Phenyl steht, das gegebenenfalls bis zu 3-fach gleich oder verschieden durch Hydroxy, Halogen oder geradkettiges oder verzweigtes Alkyl mit bis zu 5 Kohlenstoffatomen substituiert ist,

und deren Salze.

**2.** Benzyloxy-substituierte Phenylglycinolamide der Formel nach Anspruch 1 in welcher

A für Cyclopentyl, Cyclohexyl, Cyclopentenyl, Cyclohexenyl oder für Pyridyl, Phenyl oder Furyl steht, wobei die oben aufgeführten Ringe gegebenenfalls bis zu 5-fach gleich oder verschieden durch Phenyl, Pyridyl, Fluor, Chlor, Brom, Cyano, Nitro, Hydroxy, Carboxyl, durch geradkettiges oder verzweigtes Alkyl, Alkoxy, Alkoxycarbonyl, Polyfluoralkyl oder Polyfluoralkoxy mit jeweils bis zu 3 Kohlenstoffatomen oder durch eine Gruppe der Formel $-SO_2R^5$, $-NR^6R^7$ oder $-CO-NR^8R^9$ substituiert sind,
worin

$R^5$ Phenyl, Methyl oder Ethyl bedeutet,

$R^6$, $R^7$, $R^8$ und $R^9$ gleich oder verschieden sind und Wasserstoff oder geradkettiges oder verzweigtes Alkyl mit bis zu 8 Kohlenstoffatomen bedeuten,

oder

$R^8$ und/oder $R^9$ Cyclopropyl, Cyclopentyl oder Cyclohexyl bedeuten, oder Benzyl oder Phenyl bedeuten, die gegebenenfalls durch Fluor, Chlor, Brom, Nitro, Hydroxy oder durch geradkettiges oder verzweigtes Alkyl oder Alkoxy mit jeweils bis zu 3 Kohlenstoffatomen substituiert sind,

oder

$R^8$ und $R^9$ gemeinsam mit dem Stickstoffatom einen Morpholinyl-, Pyrrolidinyl-, Pyridyl- oder Piperidinylring bilden,

X für eine Bindung oder für die >C=O-Gruppe steht,

D und E gleich oder verschieden sind und für Wasserstoff, Cyclopropyl, Cyclopentyl, Cyclohexyl, Azido, Hydroxy, Fluor, Chlor, Brom, geradkettiges oder verzweigtes Alkyl, Alkoxy oder Alkenyl mit jeweils bis zu 3 Kohlenstoffatomen stehen,

$R^1$ für Cyclopropyl, Cyclobutyl, Cyclopentyl, Cyclohexyl oder Cycloheptyl steht, oder für geradkettiges oder verzweigtes Alkyl mit bis zu 8 Kohlenstoffatomen steht,

$R^2$ für Wasserstoff, oder für geradkettiges oder verzweigtes Alkyl mit bis zu 3 Kohlenstoffatomen steht,

$R^3$ für Wasserstoff oder für die $-CH_2-OH$-Gruppe steht,

$R^4$ für Phenyl steht, das gegebenenfalls bis zu 2-fach gleich oder verschieden durch Hydroxy, Fluor, Chlor, Brom oder geradkettiges oder verzweigtes Alkyl mit bis zu 4 Kohlenstoffatomen substituiert ist,

und deren Salze.

**3.** Benzyloxy-substituierte Phenylglycinolamide der Formel nach Anspruch 1 in welcher

A für Cyclopentyl, Cyclohexyl, Cyclopentenyl, Cyclohexenyl oder für Phenyl oder Pyridyl steht, wobei die oben aufgeführten Ringe gegebenenfalls bis zu 3-fach gleich oder verschieden durch Fluor, Chlor, Brom, Cyano, Nitro, Hydroxy, durch geradkettiges oder verzweigtes Alkyl, Alkoxy, Alkoxycarbonyl mit jeweils bis zu 3 Kohlenstoffatomen, Trifluormethyl, Trifluormethoxy, Carboxyl, oder durch eine Gruppe der Formel $-SO_2R^5$, $-NR^6R^7$ oder $-CO-NR^8R^9$ substituiert sind, worin

$R^5$ Phenyl oder Methyl bedeutet,

$R^6$, $R^7$, $R^8$ und $R^9$ gleich oder verschieden sind und Wasserstoff oder geradkettiges oder verzweigtes Alkyl mit bis zu 6 Kohlenstoffatomen bedeuten,

oder

$R^8$ und/oder $R^9$ Cyclopropyl, Cyclopentyl oder Cyclohexyl bedeuten, oder Benzyl oder Phenyl bedeuten, die gegebenenfalls durch Fluor, Chlor, Brom, Nitro, Hydroxy, Methyl oder Methoxy substituiert sind,

oder

$R^8$ und $R^9$ gemeinsam mit dem Stickstoffatom einen Morpholinyl-, Pyrrolidinyl- oder Piperidinylring bilden,

X für eine Bindung oder für die $>C=O$-Gruppe steht,

D und E gleich oder verschieden sind und für Wasserstoff, Fluor, Chlor oder Brom stehen,

$R^1$ für Cyclopropyl, Cyclobutyl, Cyclopentyl, Cyclohexyl oder Cycloheptyl steht, oder für geradkettiges oder verzweigtes Alkyl mit bis zu 8 Kohlenstoffatomen steht,

$R^2$ für Wasserstoff steht,

$R^3$ für Wasserstoff oder für die $-CH_2-OH$-Gruppe steht,

$R^4$ für Phenyl steht, das gegebenenfalls bis zu 2-fach gleich oder verschieden durch Hydroxy, Fluor, Chlor, Brom oder geradkettiges oder verzweigtes Alkyl mit bis zu 3 Kohlenstoffatomen substituiert ist,

und deren Salze.

**4.** Benzyloxy-substituierte Phenylglycinolamide der Formel nach Anspruch 1 in welcher

D und E für Wasserstoff stehen

und

$R^1$ für Cyclopentyl, Cyclohexyl oder Cycloheptyl steht.

**5.** Benzyloxy-substituierte Phenylglycinolamide nach Anspruch 1 bis 4 als Arzneimittel.

**6.** Verfahren zur Herstellung von Benzyloxy-substituierten Phenylglycinolamiden nach Anspruch 1 bis 4, dadurch gekennzeichnet, daß man Carbonsäuren der allgemeinen Formel (II),

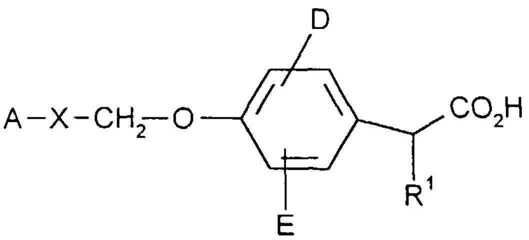 (II)

in welcher

A, D, E, X und $R^1$ die angegebene Bedeutung haben,

gegebenenfalls unter vorgeschalteter Aktivierung der Carbonsäurefunktion mit Verbindungen der allgemeinen Formel (III)

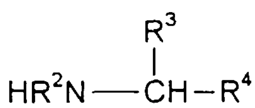 (III)

in welcher

$R^2$, $R^3$ und $R^4$ die angegebene Bedeutung haben,

gegebenenfalls unter Schutzgasatmosphäre, gegebenenfalls in inerten Lösemitteln, in Anwesenheit einer Base und/oder Hilfsmittels umsetzt.

**7.** Arzneimittel enthaltend mindestens ein Benzyloxy-substituiertes Phenylglycinolamid nach Anspruch 1 bis 4 sowie ein pharmakologisch unbedenkliches Formulierungshilfsmittel.

**8.** Arzneimittel nach Anspruch 7 zur Behandlung von Atherosklerose.

**9.** Verwendung von Benzyloxy-substituierten Phenylglycinolamiden nach Anspruch 1 bis 4 zur Herstellung von Arzneimitteln.

**10.** Verwendung von Benzyloxy-substituierten Phenylglycinolamiden nach Anspruch 1 bis 4 zur Herstellung von Arzneimitteln zur Behandlung von Atherosklerose.

**11.** Verwendung von Benzyloxy-substituierten Phenylglycinolamiden nach Anspruch 1 bis 4 zur Herstellung von Arzneimitteln zur Behandlung von Adipositas.

## Claims

**1.** Benzyloxy-substituted phenylglycinolamides of the general formula (I)

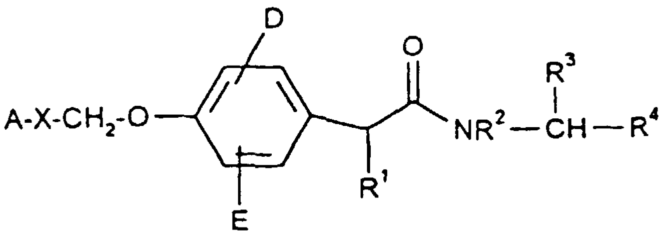 (I)

in which

A represents a 4- to 8-membered, saturated or partially unsaturated carbocycle, or represents phenyl, or represents a 5- to 6-membered aromatic heterocycle having up to 3 heteroatoms from the series S, N and/or O, the abovementioned ring systems optionally being substituted up to 5 times identically or differently by phenyl, pyridyl, carboxyl, cyano, carboxyl, halogen, nitro, hydroxyl, by straight-chain or branched alkyl, alkoxy, alkoxycarbonyl, polyfluoroalkyl or polyfluoroalkoxy each having up to 6 carbon atoms or by a group of the formula $-SO_2R^5$, $-NR^6R^7$ or $-CO-NR^8R^9$, in which

$R^5$ denotes phenyl or straight-chain or branched alkyl having up to 3 carbon atoms,

$R^6$, $R^7$, $R^8$ and $R^9$ are identical or different and denote hydrogen or straight-chain or branched alkyl having up to 10 carbon atoms,

or

$R^8$ and $R^9$ denote cycloalkyl having 3 to 6 carbon atoms, or denote benzyl or phenyl, each of which is optionally substituted by halogen, nitro, hydroxyl or by straight-chain or branched alkyl or alkoxy each having up to 4 carbon atoms,

or

$R^8$ and $R^9$, together with the nitrogen atom, form a 5- to 7-membered, saturated or unsaturated heterocycle which can optionally contain a further heteroatom from the series S, N and/ or O,

X represents a bond or the $>C=O$ group,

D and E are identical or different and represent hydrogen, cycloalkyl having 3 to 8 carbon atoms, azido, hydroxyl, halogen, straight-chain or branched alkyl, alkoxy or alkenyl each having up to 6 carbon atoms,

$R^1$ represents cycloalkyl having 3 to 8 carbon atoms, or represents straight-chain or branched alkyl having up to 10 carbon atoms,

$R^2$ represents hydrogen or represents straight-chain or branched alkyl having up to 4 carbon atoms,

$R^3$ represents hydrogen or the $-CH_2-OH$ group,

$R^4$ represents phenyl which is optionally substituted up to 3 times identically or differently by hydroxyl, halogen or straight-chain or branched alkyl having up to 5 carbon atoms,

and their salts.

**2.** Benzyloxy-substituted phenylglycinolamides of the formula according to Claim 1 in which

A represents cyclopentyl, cyclohexyl, cyclopentenyl, cyclohexenyl, or represents pyridyl, phenyl or furyl, the abovementioned rings optionally being substituted up to 5 times identically or differently by phenyl, pyridyl, fluorine, chlorine, bromine, cyano, nitro, hydroxyl, carboxyl, by straight-chain or branched alkyl, alkoxy, alkoxycarbonyl, polyfluoroalkyl or polyfluoroalkoxy each having up to 3 carbon atoms or by a group of the formula $-SO_2R^5$, $-NR^6R^7$ or $-CO-NR^8R^9$,
in which

$R^5$ denotes phenyl, methyl or ethyl,

$R^6$, $R^7$, $R^8$ and $R^9$ are identical or different and denote hydrogen or straight-chain or branched alkyl having up to 8 carbon atoms,

or

$R^8$ and/or $R^9$ denote cyclopropyl, cyclopentyl or cyclohexyl, or denote benzyl or phenyl, each of which is optionally substituted by fluorine, chlorine, bromine, nitro, hydroxyl or by straight-chain or branched alkyl or alkoxy each having up to 3 carbon atoms,

or

$R^8$ and $R^9$, together with the nitrogen atom, form a morpholinyl, pyrrolidinyl, pyridyl or piperidinyl ring,

X represents a bond or the $>C=O$ group,

D and E are identical or different and represent hydrogen, cyclopropyl, cyclopentyl, cyclohexyl, azido, hydroxyl, fluorine, chlorine, bromine, straight-chain or branched alkyl, alkoxy or alkenyl each having up to 3 carbon atoms,

$R^1$ represents cyclopropyl, cyclobutyl, cyclopentyl, cyclohexyl or cycloheptyl,
or
represents straight-chain or branched alkyl having up to 8 carbon atoms,

$R^2$ represents hydrogen, or straight-chain or branched alkyl having up to 3 carbon atoms,

$R^3$ represents hydrogen or the $-CH_2-OH$ group,

$R^4$ represents phenyl which is optionally substituted up to 2 times identically or differently by hydroxyl, fluorine, chlorine, bromine or straight-chain or branched alkyl having up to 4 carbon atoms,

and their salts.

**3.** Benzyloxy-substituted phenylglycinolamides of the formula according to Claim 1 in which

A represents cyclopentyl, cyclohexyl, cyclopentenyl, cyclohexenyl or represents phenyl or pyridyl,
the abovementioned rings optionally being substituted up to 3 times identically or differently by fluorine, chlorine, bromine, cyano, nitro, hydroxyl, by straight-chain or branched alkyl, alkoxy, alkoxycarbonyl each having up to 3 carbon atoms, trifluoromethyl, trifluoromethoxy, carboxyl, or by a group of the formula $-SO_2R^5$, $-NR^6R^7$ or $-CO-NR^8R^9$,
in which

$R^5$ denotes phenyl or methyl,

$R^6$, $R^7$, $R^8$ and $R^9$ are identical or different and denote hydrogen or straight-chain or branched alkyl having up to 6 carbon atoms,

or

$R^8$ and/or $R^9$ denote cyclopropyl, cyclopentyl or cyclohexyl, or denote benzyl or phenyl, each of which is optionally substituted by fluorine, chlorine, bromine, nitro, hydroxyl, methyl or methoxy,

or

$R^8$ and $R^9$, together with the nitrogen atom, form a morpholinyl, pyrrolidinyl or piperidinyl ring,

X represents a bond or the >C=O group,

D and E are identical or different and represent hydrogen, fluorine, chlorine or bromine,

$R^1$ represents cyclopropyl, cyclobutyl, cyclopentyl, cyclohexyl or cycloheptyl, or represents straight-chain or branched alkyl having up to 8 carbon atoms,

$R^2$ represents hydrogen,

$R^3$ represents hydrogen or the $-CH_2-OH$ group,

$R^4$ represents phenyl which is optionally substituted up to 2 times identically or differently by hydroxyl, fluorine, chlorine, bromine or straight-chain or branched alkyl having up to 3 carbon atoms,

and their salts.

**4.** Benzyloxy-substituted phenylglycinolamides of the formula according to Claim 1 in which

D and E represent hydrogen

and

$R^1$ represents cyclopentyl, cyclohexyl or cycloheptyl.

**5.** Benzyloxy-substituted phenylglycinolamides according to Claims 1 to 4 as medicaments.

**6.** Process for the preparation of benzyloxy-substituted phenylglycinolamides according to Claims 1 to 4, characterized in that
carboxylic acids of the general formula (II)

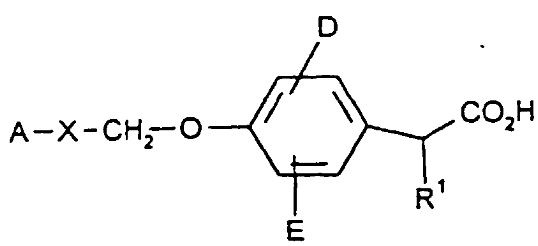

(II)

in which

A, D, E, X and $R^1$ have the meaning indicated,

are reacted, if appropriate with prior activation of the carboxylic acid function, with compounds of the general formula (III)

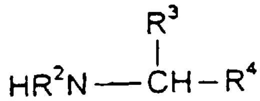

(III)

in which

$R^2$, $R^3$ and $R^4$ have the meaning indicated,

if appropriate under a protective gas atmosphere, if appropriate in inert solvents, in the presence of a base and/or auxiliary.

**7.** Medicaments containing at least one benzyloxy-substituted phenyl-glycinolamide according to Claims 1 to 4 and a pharmacologically acceptable formulation auxiliary.

**8.** Medicaments according to Claim 7 for the treatment of atherosclerosis.

**9.** Use of benzyloxy-substituted phenylglycinolamides according to Claims 1 to 4 for the production of medicaments.

**10.** Use ofbenzyloxy-substituted phenylglycinolamides according to Claims 1 to 4 for the production of medicaments for the treatment of atherosclerosis.

**11.** Use of benzyloxy-substituted phenylglycinolamides according to Claims 1 to 4 for the production of medicaments for the treatment of adipositas.

**Revendications**

**1.** Phénylglycinolamides à substituant benzyloxy, de formule générale (I) :

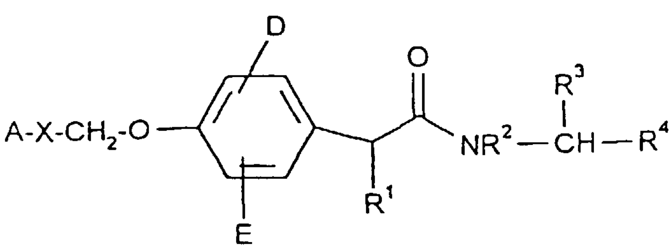

(I)

dans laquelle

A représente un carbocycle de 4 à 8 atomes, saturé ou partiellement insaturé, ou un groupe phényle, ou un hétérocycle aromatique pentagonal ou hexagonal ayant jusqu'à 3 hétéroatomes de la série S, N et/ou O, les systèmes cycliques indiqués ci- dessus étant éventuellement substitués jusqu'à 5 fois identiques ou différentes par un groupe phényle, pyridyle, carboxyle, cyano, carboxyle, halogéno, nitro, hydroxy, par un groupe alkyle, alkoxy, alkoxycarbonyle, polyfluoralkyle ou polyfluoralkoxy, linéaire ou ramifié, ayant chacun jusqu'à 6 atomes de carbone, ou par un groupe de formule $-SO_2R^5$, $-NR^6R^7$ ou $-CO-NR^8R^9$, où

$R^5$ est un groupe phényle ou un groupe alkyle linéaire ou ramifié ayant jusqu'à 3 atomes de carbone,

$R^6$, $R^7$, $R^8$ et $R^9$ sont identiques ou différents et représentent l'hydrogène ou un groupe alkyle linéaire ou ramifié ayant jusqu'à 10 atomes de carbone,

ou bien

$R^8$ et/ ou $R^9$ représentent un groupe cycloalkyle ayant 3 à 6 atomes de carbone, ou représentent des groupes benzyle ou phényle qui sont substitués le cas échéant par un halogène, un groupe nitro, hydroxy ou par un groupe alkyle ou alkoxy linéaire ou ramifié ayant dans chaque cas jusqu'à 4 atomes de carbone,

ou bien

$R^8$ et $R^9$ forment conjointement avec l'atome d'azote un hétérocycle pentagonal à heptagonal, saturé ou non saturé, qui peut éventuellement contenir un autre hétéroatome de la série S, N et/ ou O,

X représente une liaison ou le groupe $>C=O$
D et E sont identiques ou différents et représentent l'hydrogène, un groupe cycloalkyle ayant 3 à 8 atomes de carbone, azido, hydroxy, halogéno, un groupe alkyle, alkoxy ou alcényle, linéaire ou ramifié, ayant dans chaque cas jusqu'à 6 atomes de carbone,
$R^1$ est un groupe cycloalkyle de 3 à 8 atomes de carbone, ou représente un groupe alkyle linéaire ou ramifié ayant jusqu'à 10 atomes de carbone,
$R^2$ est l'hydrogène ou un groupe alkyle linéaire ou ramifié ayant jusqu'à 4 atomes de carbone,
$R^3$ représente l'hydrogène ou le groupe $-CH_2-OH$,
$R^4$ est un groupe phényle qui est substitué le cas échéant jusqu'à trois fois identiques ou différentes par un radical hydroxy, un halogène ou un radical alkyle linéaire ou ramifié, ayant jusqu'à 5 atomes de carbone,

et leurs sels.

**2.** Phénylglycinolamides à substituant benzyloxy de formule suivant la revendication 1, dans laquelle

A représente un groupe cyclopentyle, cyclohexyle, cyclopentényle, cyclohexényle, ou un groupe pyridyle, phényle ou furyle,
les noyaux indiqués ci- dessus étant éventuellement substitués jusqu'à 5 fois identiques ou différentes par un groupe phényle, pyridyle, du fluor, du chlore, du brome, un groupe cyano, nitro, hydroxy, carboxyle, par un groupe alkyle, alkoxy, alkoxycarbonyle, polyfluoralkyle ou polyfluoralkoxy, linéaire ou ramifié, ayant dans chaque cas jusqu'à 3 atomes de carbone, ou par un groupe de formule $-SO_2R^5$, $-NR^6R^7$ ou $-CO-NR^8R^9$,
où

$R^5$ est un groupe phényle, méthyle ou éthyle,
$R^6$, $R^7$, $R^8$ et $R^9$ sont identiques ou différents et représentent l'hydrogène ou un groupe alkyle linéaire ou ramifié ayant jusqu'à 8 atomes de carbone,

ou bien

$R^8$ et/ ou $R^9$ représentent un groupe cyclopropyle, cyclopentyle ou cyclohexyle, ou représentent un groupe benzyle ou un groupe phényle qui sont substitués le cas échéant par du fluor, du chlore, du brome, un radical nitro, hydroxy, ou par un radical alkyle ou alkoxy linéaire ou ramifié ayant chacun jusqu'à 3 atomes de carbone,

ou bien

$R^8$ et $R^9$ forment conjointement avec l'atome d'azote un noyau morpholinyle, pyrrolidinyle, pyridyle ou pipéridinyle,

X représente une liaison ou le groupe $>C=O$

D et E sont identiques ou différents et représentent l'hydrogène, un groupe cyclopropyle, cyclopentyle, cyclohexyle, azido, hydroxy, du fluor, du chlore, du brome, un groupe alkyle, alkoxy ou alcényle, linéaire ou ramifié, ayant dans chaque cas jusqu'à 3 atomes de carbone,
$R^1$ est un groupe cyclopropyle, cyclobutyle, cyclopentyle, cyclohexyle ou cycloheptyle, ou bien un groupe alkyle linéaire ou ramifié ayant jusqu'à 8 atomes de carbone,
$R^2$ représente l'hydrogène ou un groupe alkyle linéaire ou ramifié ayant jusqu'à 3 atomes de carbone,
$R^3$ représente l'hydrogène ou le groupe $-CH_2-OH$,
$R^4$ est un groupe phényle qui est substitué le cas échéant jusqu'à deux fois identiques ou différentes par un radical hydroxy, fluoro, chloro, bromo, ou par un radical alkyle linéaire ou ramifié ayant jusqu'à 4 atomes de carbone,

et leurs sels.

**3.** Phénylglycinolamides à substituant benzyloxy de formule suivant la revendication 1, dans laquelle

A représente un groupe cyclopentyle, cyclohexyle, cyclopentényle, cyclohexényle, ou un groupe phényle ou pyridyle,
les noyaux indiqués ci- dessus étant éventuellement substitués jusqu'à 3 fois identiques ou différentes par du fluor, du chlore, du brome, un groupe cyano, nitro, hydroxy, par un radical alkyle, alkoxy, alkoxycarbonyle linéaire ou ramifié, ayant dans chaque cas jusqu'à 3 atomes de carbone, un radical trifluorométhyle, trifluorométhoxy, carboxyle, ou par un groupe de formule $-SO_2R^5$, $-NR^6R^7$ ou $-CO-NR^8R^9$, où

$R^5$ représente un groupe phényle ou méthyle,
$R^6$, $R^7$, $R^8$ et $R^9$ sont identiques ou différents et représentent l'hydrogène ou un groupe alkyle linéaire ou ramifié ayant jusqu'à 6 atomes de carbone,

ou bien

$R^8$ et/ ou $R^9$ représentent des groupes cyclopropyle, cyclopentyle ou cyclohexyle, ou représentent un groupe benzyle ou un groupe phényle qui sont substitués le cas échéant par du fluor, du chlore, du brome, un radical nitro, hydroxy, méthyle ou méthoxy,

ou bien

$R^8$ et $R^9$ forment conjointement avec l'atome d'azote un noyau morpholinyle, pyrrolidinyle ou pipéridinyle,

X représente une liaison ou le groupe $>C=O$
D et E sont identiques ou différents et représentent l'hydrogène, le fluor, le chlore ou le brome,
$R^1$ est un groupe cyclopropyle, cyclobutyle, cyclopentyle, cyclohexyle ou cycloheptyle, ou bien un groupe alkyle linéaire ou ramifié ayant jusqu'à 8 atomes de carbone,
$R^2$ représente l'hydrogène,
$R^3$ représente l'hydrogène ou le groupe $-CH_2-OH$,
$R^4$ est un groupe phényle qui est substitué le cas échéant jusqu'à deux fois identiques ou différentes par un radical hydroxy, fluoro, chloro, bromo, ou par un radical alkyle linéaire ou ramifié, ayant jusqu'à 3 atomes de carbone,

et leurs sels.

**4.** Phénylglycinolamides à substituant benzyloxy de formule suivant la revendication 1, dans laquelle

D et E représentent l'hydrogène,

et

$R^1$ est un groupe cyclopentyle, cyclohexyle ou cycloheptyle.

**5.** Phénylglycinolamides à substituant benzyloxy suivant les revendications 1 à 4, utilisés comme médicaments.

**6.** Procédé de production de phénylglycinolamides à substituant benzyloxy suivant les revendications 1 à 4, caractérisé en ce que l'on fait réagir les acides carboxyliques de formule générale (II) :

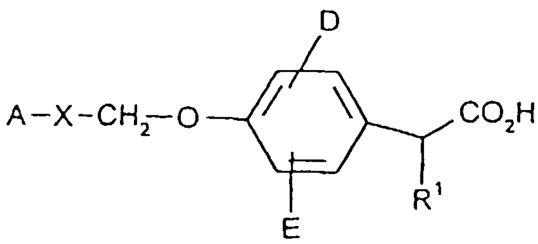
(II)

dans laquelle
A, D, E, X et $R^1$ ont la définition indiquée,
éventuellement avec activation préalable de la fonction acide carboxylique,
avec des composés de formule générale (III) :

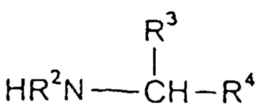
(III)

dans laquelle
$R^2$, $R^3$ et $R^4$ ont la définition indiquée ci- dessus,
éventuellement sous atmosphère de gaz protecteur, le cas échéant dans des solvants inertes, en présence d'une base et/ ou d'une substance auxiliaire.

**7.** Médicament contenant au moins un phénylglycinolamide à substituant benzyloxy suivant les revendications 1 à 4, ainsi qu'un auxiliaire de formulation acceptable du point de vue pharmacologique.

**8.** Médicament suivant la revendication 7, destiné au traitement de l'athérosclérose.

**9.** Utilisation de phénylglycinolamides à substituant benzyloxy suivant les revendications 1 à 4, pour la préparation de médicaments.

**10.** Utilisation de phénylglycinolamides à substituant benzyloxy suivant les revendications 1 à 4, pour la préparation de médicaments destinés au traitement de l'athérosclérose.

**11.** Utilisation de phénylglycinolamides à substituant benzyloxy suivant les revendications 1 à 4, pour la préparation de médicaments destinés au traitement de l'adiposité.